(19) **Europäisches Patentamt**
**European Patent Office**
**Office européen des brevets**

(11) **EP 1 303 210 B1**

(12) **EUROPÄISCHE PATENTSCHRIFT**

(45) Veröffentlichungstag und Bekanntmachung des Hinweises auf die Patenterteilung:
**31.12.2008 Patentblatt 2009/01**

(51) Int Cl.:
*A61B 3/11* *(2006.01)*      *A61B 3/113* *(2006.01)*

(21) Anmeldenummer: **02716785.7**

(22) Anmeldetag: **16.02.2002**

(86) Internationale Anmeldenummer:
**PCT/EP2002/001675**

(87) Internationale Veröffentlichungsnummer:
**WO 2002/065899 (29.08.2002 Gazette 2002/35)**

(54) **VERFAHREN ZUR ERMITTLUNG VON ABSTÄNDEN AM VORDEREN AUGENABSCHNITT**

METHOD FOR DETERMINING DISTANCES IN THE ANTERIOR OCULAR SEGMENT

PROCEDE PERMETTANT DE DETERMINER DES DISTANCES DANS LA SECTION AVANT DE L'OEIL

(84) Benannte Vertragsstaaten:
**AT BE CH CY DE DK ES FI FR GB GR IE IT LI LU MC NL PT SE TR**

(30) Priorität: **21.02.2001 DE 10108797**

(43) Veröffentlichungstag der Anmeldung:
**23.04.2003 Patentblatt 2003/17**

(73) Patentinhaber: **Carl Zeiss Meditec AG**
**07745 Jena (DE)**

(72) Erfinder:
• **BERGNER, Roland**
**07745 Jena (DE)**
• **BARTH, Roland**
**07743 Jena (DE)**
• **DOERING, Axel**
**07745 Jena (DE)**
• **BEHRENDT, Frank**
**07743 Jena (DE)**
• **VOIGT, Klaus-Ditmar**
**07745 Jena (DE)**

(74) Vertreter: **Geyer, Fehners & Partner**
**Patentanwälte**
**Perhamerstraße 31**
**80687 München (DE)**

(56) Entgegenhaltungen:
EP-A- 0 821 912      WO-A-00/33729
DE-A- 19 618 883      US-A- 5 231 674
US-A- 5 327 191      US-A- 5 469 234
US-A- 5 474 548      US-A- 5 790 235

**Beschreibung**

Die Erfindung bezicht sich auf ein Verfahren zur Ermittlung von Abständen am vorderen Augenabschnitt, insbesondere des Irisdurchmessers. Für die IOL( Intraokularlinse) -Berechnungsformel "Holladay 2" ("Intraocular Lens Power Calculations for the Refractive Surgeon", Jack T. Holladay, in: Operative Techniques in Cataract and Refractive Surgery, Vol. 1, No 3 (September), 1998: pp 105-117) , mit welcher die Stärke einer in das menschliche Auge zu implantierenden Intraokularlinse (IOL) berechnet werden kann, sowie für die Auswahl spezieller IOL Typen (ICL u.a.) wird als ein Eingarigsparameter der sogenannte "horizontale-white-to-white-Abstand" (hor-w-t-w) benötigt. Das ist der horizontale Durchmesser der Iris.

Für die Homhautchirurgie zur Beseitigung von Fehlsichtigkeiten des menschlichen Auges (PRK, LASIK) ist es für den Operateur auch von Interesse, an welchem Punkt die Sehachse des Patienten die Hornhaut durchstößt. Danach kann die Laserabtragung von diesem Punkt aus präziser erfolgen, als mit der bisherigen Annahme, welche vom geometrischen Mittelpunkt der Hornhaut ausgeht.

Für die interferometrische Längenmessung der Hornhautdicke,

Vorderkammertiefe und Linsendicke am menschlichen Auge mittels PCI ist es notwendig, das Auge entlang seiner hypothetischen optischen Achse vor dem

Meßgerät voreinzustellen, im Unterschied zur Achslängenmessung, bei welcher das Auge entlang der tatsächlichen Sehachse positioniert werden muß.

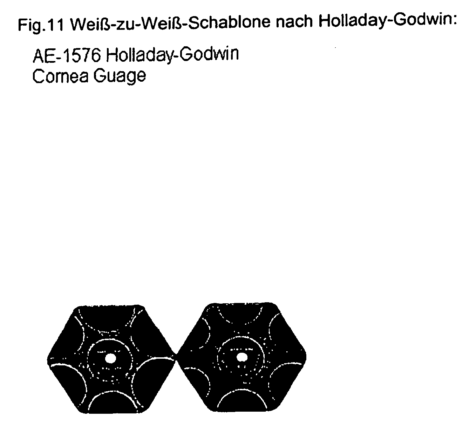

Zur Ermittlung des "hor-w-t-w" werden bisher Lineale bzw. Schablonen verwendet (Fig.11 und http: //www.asico.com/1576.htm), welche dem Patientenauge vorgehalten werden und mit denen der Durchmesser der Iris durch Peilen abgelesen wird. Dieses Verfahren ist störanfällig gegenüber Parallaxe beim Beobachten, und die bisher verwendeten Schablonen haben eine Abstufung von 0,5 mm. Damit ist nur eine eingeschränkte Genauigkeit möglich. Eine andere bekannte Lösung sind Meßokulare, welche an Spaltlampengeräten als Zubehör zum Einsatz kommen (Gebrauchsanweisung Spaltlampe 30 SUM Druckschriften Nr: G 30-114-d (MA XI/79), Carl Zeiss, D-7082 Oberkochen, Seite 38). Diese verhindern zwar Parallaxenlehler, es muß aber an einer Skale der Durchmesserwert abgelesen werden.

Weiter sind invasive Meßmittel in Form von mechanischen Meßschiebern bekannt, welche durch einen Schnitt in der Sklera in die Vorderkammer eingeführt werden (z.B. US 4319564).

Weiter sind sogenannte Gonioskope bekannt, welche auf das Auge aufgesetzt werden, Skalen auf die Iris projizieren und durch Lupen kann der Irisdurchmesser abgelesen werden (z.B. US 4398812).

Vorrichtungen, welche den Durchmesser der Pupille messen, sind sogenannte Pupillometer (z.B. EP 0550673). Diese messen aber nicht den Durchmesser der Iris.

Für die Bestimmung des Durchtrittspunktes der Sehachse durch die Hornhaut sind keine Vorrichtungen bekannt. In der Kameraindustrie werden lediglich Verfahren verwendet, welche die Blickrichtung eines menschlichen Auges detektieren; die Ausgangssignale solcher Anordnungen dienen zum Beispiel der Steuerung von Autofokusmechanismen in Fotokameras (z.B. US 5291234, US 5469234) bzw sie werden in sogenannten Eyetrackern (z.B. De-A-19618883) eingesetzt. Diese Geräte überwachen die Augen- bzw. Blickbewegungen.

Zur Voreinstellung des Auges entlang der optischen Achse sind ebenfalls keine Vorrichtungen bekannt; es wird durch Umfixieren des Auges bzw. Scannen des Meßstrahles relativ ungezielt versucht, die richtige Positionierung des Auges entlang der optischen Achse durch Probieren zu finden.

Aus WO 00/33729 der Anmelderin ist eine Anordnung und ein Verfahren bekannt, um mittels eines einzigen Gerätes zur Berechnung der optischen Wirkung einer Introkularlinse berührungslos die Achslänge,

Hornhautkrümmung und Vorderkammertiefe des Auges zu bestimmen. Das Auge wird über sichtbare oder IR -LED allgemein beleuchtet, wobei deren Reflexbilder mittels der CCD-Kamera erfaßt und dargestellt werden. Weiter ist ein Fixierlicht vorgesehen, damit der Proband die Augenpupille in Richtung der optischen Achse ausrichtet, wobei der Reflex des Fixierlichtes ebenfalls von der CCD - Kamera erfaßt wird.

Aufgabe der Erfindung ist es, ein Verfahren zu schaffen, welches bedienerunabhängig eine höhere Genauigkeit der hor-w-t-w Bestimmung zuläßt . Die Aufgabe wird erfindungsgemäß durch die Merkmale des Anspruchs 1 gelöst. Bevorzugte Weiterbildungen sind Gegenstand der abhängigen Ansprüche.

Überraschend realisiert die Erfindung auch eine praktikable Möglichkeit, den Durchtrittspunkt der Sehachse durch die Hornhaut in Bezug auf den Mittelpunkt der Pupille und / oder Iris zu beschreiben und aus der Position dieses Durchtrittspunktes und der geometrischen Mitte der Hornhaut eine präzisere Voreinstellung eines interferometrischen Längenmeßgerätes entlang der optischen Achse des Auges vornehmen zu können.

Bisher war die Lage der Sehachse dem Gerätebediener unbekannt; deshalb wurde der Patient mit geeigneten Mitteln (Fixierlicht im Gerät oder außerhalb des Gerätes für das Partnerauge) aufgefordert, umzufixieren. Im Anschluß wurde ein Meßvorgang ausgelöst, der aber nur erfolgreich ist, wenn die Messung entlang der optischen Achse erfolgte. Das bedeutet, daß erst im Anschluß an den Meßvorgang klar wird, ob diese Achse getroffen wurde oder nicht.

In Schritten von 1° wird das Fixierlicht bewegt; es können recht viele unbrauchbare Meßvorgänge erforderlich werden, bis die Stelle erreicht wird, an der die interferometrische Messung erfolgreich ist.

Für die ophthalmologische Routine ist diese Vorgehensweise nicht akzeptabel.

Die Erfindung wird nachstehend anhand der schematischen Zeichnungen in einem Ausführungsbeispiel näher erläutert.

Es zeigen:

Fig.1: Den schematischen Ablauf des erfindungsgemäßen Verfahrens,
Fig.2: Den Algorithmus zur Grobdetektion der Pupille,
Fig.3: Eine Darstellung zur Grauwertanalyse / Mittelpunktsermittlung,
Fig.4: Eine Darstellung der Kantenanalyse,
Fig.5: Den schematischen Ablauf der Kantenanalyse,
Fig.6: Die Detektion und Ermittlung der Lage des Fixierpunktes,
Fig.7: Den schematischen Ablauf der Plausibilitätskontrolle,
Fig.8: Den Beleuchtungs/Detektionsstrahlengang,
Fig.9: Ein Überblicksbild des zu vermessenden Auges,
Fig.10: Die vergrößerte Darstellung des Zentrums in Fig.9, und
Fig.11: Eine Weiss zu Weiss Schablone nach Holladay-Godwin.

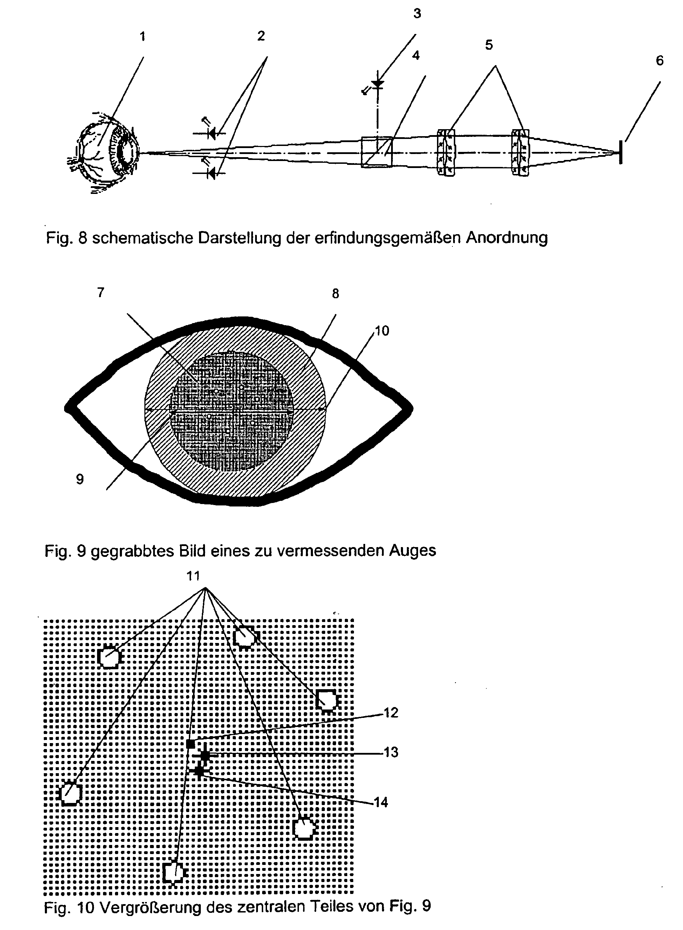

Das Probandenauge 1 wird gemäß Fig. 8 durch vorzugsweise infrarot strahlende, kreisförmig um die optische Achse angeordnete Lichtquellen 2, wie in WO 00/33729 (z.B. LED) beschrieben beleuchtet. Im Beobachtungssystem wird koaxial zum Beobachtungsstrahlengang eine Lichtquelle 3 über einen Strahlteiler 4 eingeblendet, welche sichtbares Licht aussendet (z.B. LED oder Laserdiode), auf welche der Proband fixiert.

Das Bild des Auges wird über ein telezentrisches Abbildungssystem 5 auf einen Bildsensor 6, vorzugsweise eine CCD - Kamera, der mit einer nicht dargestellten Steuer- und Auswerteeinheit verbunden ist, abgebildet. Das Videosignal der Kamera wird auf einem (nicht dargestellten) Monitor oder LC Display dargestellt.

Der Bediener kann sich durch die Beleuchtung 2 während der gesamten Zeit der Einjustierung und der Vermessung des Probanden davon überzeugen, daß der Proband richtig fixiert und somit das Meßergebnis unverfälscht ist.

Die Abbildung des Probandenauges mit den relevanten Bildausschnitten wird telezentrisch durchgeführt, um den Einfluß der Probandenjustierung zu minimieren.

Das BAS - Signal der CCD - Kamera wird nach der korrekten Einjustierung des Patientenauges und nach Auslösung durch den Bediener mittels Frame

Grabber in den Speicher eines Rechners in die Auswerteeinheit übemommen. In Fig.9 ist ein derartiges Bild schematisch dargestellt, mit Pupille 7, Pupillendurchmmesser 9 sowie Iris 8 und Irisdurchmesser 10.

Fig.10 zeigt einen vergrößerten Abschnitt der Pupille mit Reflexpunkten 11 der Beleuchtung , dem Bild des Fixierlichtes 12 , Irismittelpunkt 13 und

Pupillenmittelpunkt 14.

Mit Mitteln der Bildverarbeitung werden die Abstände im Bild ermittelt, aus denen über den Abbildungsmaßstab der Beobachtungsoptik folgende Größen berechnet werden können:

- der Durchmesser und der Mittelpunkt der Iris,
- der Durchmesser und der Mittelpunkt der Pupille,
- die x- und y-Koordinaten des Hornhautbildes des Fixierlichtes (1. Purkinjebild) in Bezug auf den Mittelpunkt der Iris und
- die x- und y-Koordinaten des Hornhautbildes des Fixierlichtes (1. Purkinjebild) in Bezug auf den Mittelpunkt der Pupille.

Da die realen Formen von Iris und Pupille des menschlichen Auges nicht notwendigerweise Kreise sein müssen, können in einer weiteren Ausführungsform auch Ellipsen mit ihren Parametern, Halbachsen und Brennpunkte bestimmt werden.

Die Meßgrößen lassen sich bei geeigneter Wahl des Abbildungsmaßstabes der Abbildungsoptik 5 mit einer rechnerischen Genauigkeit von $< \pm 0,01$ mm bestimmen.

Der Durchmesser der Iris ergibt den horizontalen white-to-white Abstand: white-to-white [in mm] = $\varnothing_{iris}$ [in Pixel] / Anzahl der Pixel pro mm

Die x-und y-Koordinaten des Purkinjebildes des Fixierlichtes ergeben den Durchstoßungspunkt der Sehachse durch die Hornhaut unter der Voraussetzung, daß der Proband richtig fixiert, was der Bediener anhand des Video-life-Bildes auf dem LC-Display während der Messung kontrollieren kann. Sehachse und opt. Achse können bis zu 8° voneinander abweichen, da die

Fovea 3° nasal bis 8° temporal versetzt liegen kann. (Vereinfachtes schematisches Auge nach Gullstrand in Diepes, "Refraktionsbestimmung", Verlag Bode, Pforzheim, 5. Auflage 1988).

Der Winkel zwischen der Sehachse und der optischen Achse des Auges ergibt sich aus Winkelbeziehungen beispielsweise anhand des Gullstrand- Auges, in welche die gemessene Ablage (Abstand) des Bildes des Fixierpunktes vom Iris- und/oder Pupillenmittelpunkt eingeht.

Vor der interferometrischen Vermessung der vorderen Augenmedien wird die Abweichung beider Sehachsen zueinander bestimmt. Der Fixierpunkt des vorliegenden Meßsystems markiert sich entlang der Sehachse. Der Bertrag und die Richtung des Abstandes dieses Punktes zur Pupillenmitte (und oder Irismitte) wird ermittelt.

Durch einfache trigonometrische Formeln erhält man den gesuchten Winkel zwischen optischer Achse und Sehachse, beispielsweise zu

$$\alpha = \arctan(a/k) \ ,$$

mit :

$\alpha$ - Winkel zwischen Seh- und opt. Achse,

a - Abstand Fixierpunkt zur Pupillenmitte (Irismitte),

k - Abstand Knotenpunkt ( s. Literatur Diepes) zur Hornhaut minus R/2 (etwa 3,8 mm).

Entsprechend dieses Meßwertes kann vorteilhaft eine Voreinstellung der Blickrichtung des Patienten erfolgen, indem dem Patienten ein Fixierlicht unter dem errechneten Winkel $\alpha$ angeboten wird. Damit wird ein aufwendiges Suchverfahren überflüssig.

Verfahren zur Lagebestimmung von Pupille, Iris und Fixierpunkt

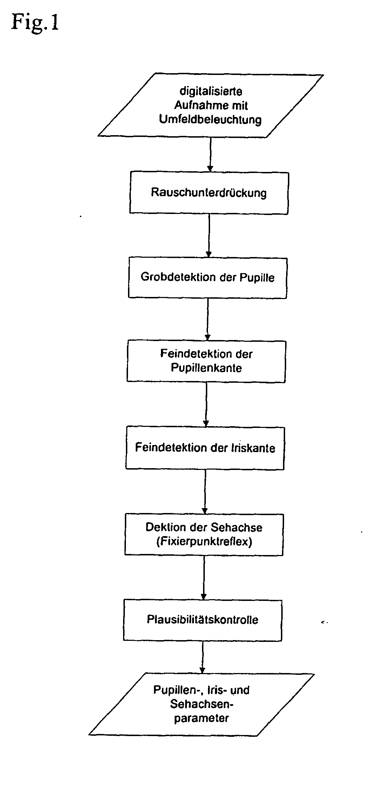

*Ablaufplan (Fig. 1):*

Als Eingabegröße der Auswertung wird eine digitalisierte Graubildaufnahme verwendet in einem Abbildungsmaßstab, der eine Erfassung der gesamten Iris erlaubt, mit eingeschalteter Umfeldbeleuchtung.

In der Auswerteeinheit werden nach Rauschunterdrückung die Objekte Pupille, Iris und Fixierpunktbild bestimmt.

Zunächst wird das Pupillenbild grob ermittelt und zur Irisdetektion herangezogen, da der Kontrast an der Iriskante in der Regel schwach ist und außerdem der Irisrand oben und unten durch Augenlider verdeckt sein kann, so daß keine kreisförmige sondern nur eine sektorförmige Erfassung möglich ist.

Bei erfolgreicher Abarbeitung werden die Parameter der Iris und der Pupille als Kreismodell (Radius, Mittelpunkt) oder als Ellipsenmodell (Hauptachsen,

Mittelpunkt) zurückgegeben. Der Fixierpunkt (d.h. der Durchtrittspunkt der Sehachse durch die Kornea) wird in seinen Koordinaten zurückgegeben, d.h., die Koordinaten stehen dem aufrufenden Programm zur Verfügung.

*Rauschunterdrückung*

Eine Kantendetektion auf der Basis von Grauwertprofilen im Originalbild führt zu großen Streuungen bei der Bestimmung der Kantenorte, die auf ein dem Bildsignal überlagertes Rauschen zurückzuführen sind. Zur Rauschunterdrückung wird ein 20x20-Medianfilter eingesetzt.

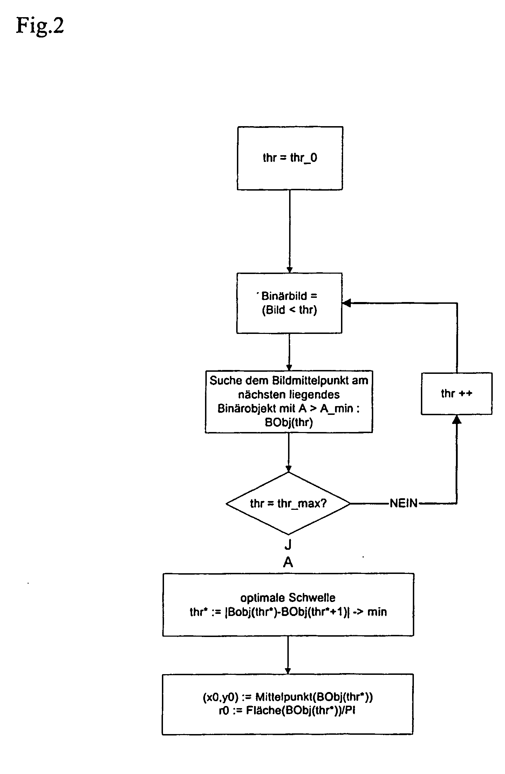

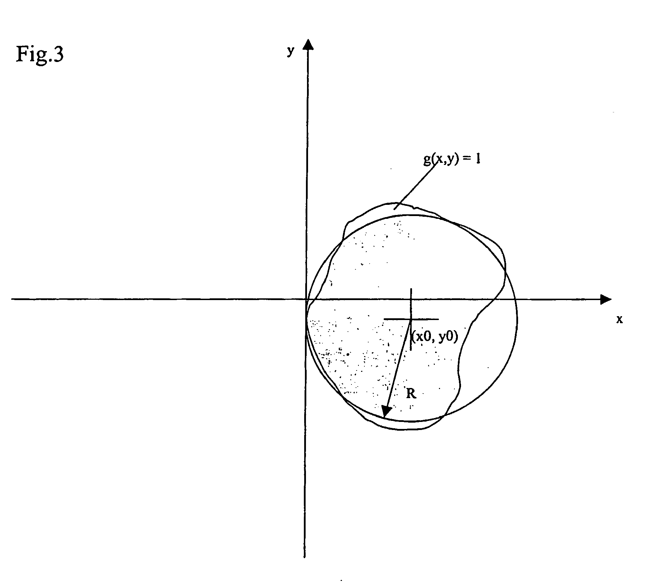

*Grobdetektion der Pupille* (*Fig.2*/*Fig.3*)

Zur groben Bestimmung der Pupillenlage wird ein Binarisierungsverfahren mit anschließender Suche nach zusammenhängenden Objekten im Binärbild verwendet.

In Fig. 3 ist eine von der CCD Kamera erfaßte unregelmäßige Grauwertverteilung g(x,y), ermittelt durch Schwellwertanalyse (Schwellwert 1), in einem X/Y-Koordinatensystem dargestellt. Von dieser Fläche wird über eine Schwerpunktanalyse der theoretische Mittelpunkt xo,yo bestimmt und ein Kreismodell/ Ellipsenmodell mit einem Radius R ermittelt. Das wird weiter unten erklärt.

Als Binarisierung wird die pixelweise Grauwerttransformation nach $b(x,y) = \begin{cases} 1 & wenn\ g(x,y) \geq thr \\ 0 & sonst \end{cases}$ verstanden, wobei gilt:

x         horizontale Koordinate eines Pixels,

y         vertikale Koordinate eines Pixels,

g(x,y)  Grauwert des Pixels an der Stelle (x,y),

thr   nicht-negativer Schwellwert.

Als Pupille wird dasjenige Binärobjekt angenommen, das eine vorgegebene Mindestgröße überschreitet und dem Bildmittelpunkt am nächsten liegt.

Ein Binarisierungsverfahren mit konstantem Schwellwert ist - wegen seiner Abhängigkeit von der Umgebungshelligkeit - nicht geeignet. Daher werden nach o.g. Verfahren Binärobjekte für eine Folge von Schwellwerten bestimmt. Als "optimaler" Schwellwert thr* wird derjenige Wert angenommen, bei dessen Inkrementierung die geringste Veränderung beim ausgewählten Binärobjekt (d.h. Lage und Größe) stattfindet. Aus dem diesem Schwellwert zugeordneten Binärobjekt werden als Grobschätzung der Pupillenlage folgende Größen bestimmt:

$$x_0 = \frac{\sum g(x,y) \cdot x}{\sum g(x,y)} \qquad \text{(Summation über alle Pixel des Bildes)}$$

$$y_0 = \frac{\sum g(x,y) \cdot y}{\sum g(x,y)}$$

$$R = \sqrt{\frac{1}{\pi} \sum g(x,y)} \qquad\qquad \textit{wobei}$$

$$g(x,y) = \begin{cases} 1 & (x,y) \in \textit{Binärobjekt} \\ 0 & \textit{sonst} \end{cases}$$

$(x_0, y_0)$  Flächenschwerpunkt des Binärobjekts (Mittelpunktskoordinaten),

R    (Fläche des Binärobjekts / $\pi$)$^{1/2}$ (geschätzter Radius) .

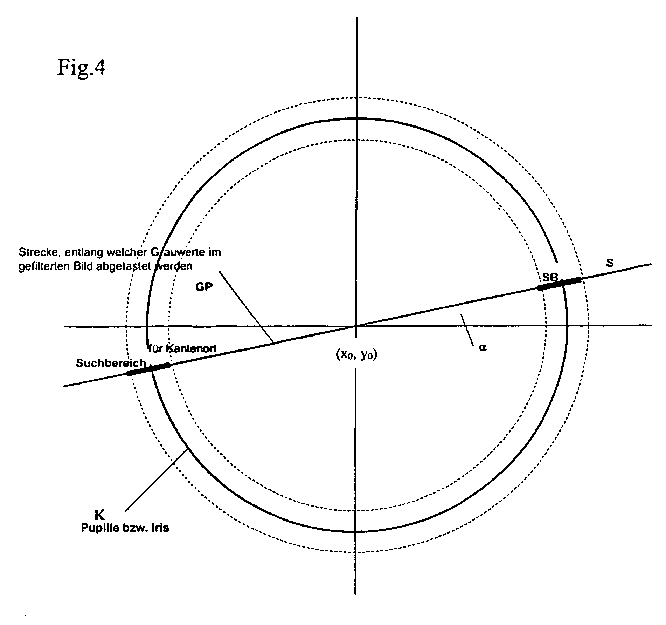

### Feindetektion von Pupille und Iris (*Fig. 4*)

Die Kantenorte für Pupille und Iris (Kante = "Rand" des Kreises/der Ellipse) werden aus Grauwertprofilen (= Abtastungen der Grauwerte des mediangefilterten Bildes entlang Strecken) durch den Mittelpunkt der grob bestimmten Pupille ermittelt (siehe Abbildung). Für die Iris wird zunächst angenommen, daß sich die Kante in einem bestimmten größeren, konzentrisch zur grob detektierten Pupille angeordneten Kreisring befindet. Der folgende Algorithmus gilt sinngemäß sowohl für die Feindetektion der Pupillen- als auch der Iriskante.

Die Abtastung erfolgt mittels Suchstrahlen (Suchrichtungen) S von xo,yo aus, wobei die Suchstrahlrichtung um einen Winkel $\alpha$ sukzessive geändert wird.

Der grobe Suchbereich SB auf dem Suchstrahl S ergibt sich durch das bereits ermittelte grobe Modell von Iris und Pupille. Die Iriskantenbestimmung erfolgt wegen möglicher Lidabdeckung nur in einem Winkelbereich um die X-Achse (2 Kreissektoren).

In diesen Profilen werden Wendepunkte durch eine geeignete Glättung und numerische Differentiation bestimmt. Hierzu sind eine Reihe von Verfahren (z.B. Savitzky A. and Golay, M.J.E. Analytical Chemistry, Vol. 36, S. 1627-39, 1964) bekannt, die sich als eindimensionale lineare Filter effizient implementieren lassen. Im allgemeinen wird eine Vielzahl von Wendepunkten entlang des Grauwertprofils gefunden. Als Kantenort wird darunter diejenige Position (x,y) ermittelt, die folgende Bedingungen erfüllt:

(a) (x,y) liegt in einem Kreisring um $(x_0,y_0)$.(Grobposition der Pupille) mit einem inneren und äußeren Radius, der jeweils für die Pupillen- und Irisdetektion in Abhängigkeit von r0 (Grobradius der Pupille) bestimmt werden kann,

(b) Die Differenz zwischen den um (x,y) im Grauwertprofil liegenden Extremwerten wird betragsmäßig maximal unter allen Positionen, die (a) erfüllen.

Damit stehen pro Grauwertprofil (d.h., pro Abtastwinkel $\alpha$) maximal je zwei Kantenorte für die Iris- und die Pupillenmodellierung zur Verfügung. Zur Ausschaltung systematischer Störungen beispielsweise durch Abdeckung der Iris oder der Pupille bei verengter Lidspalte wird der verwendete Bereich von Abtastwinkeln eingeschränkt, d.h. $\alpha_{min, \text{ Iris}} < \alpha < \alpha_{max, \text{ Iris}}$ für die Iriskantenbestimmung und analog $\alpha_{min, \text{ Pupille}} < \alpha < \alpha_{max, \text{ Pupille}}$ für die Pupillenkantenbestimmung.

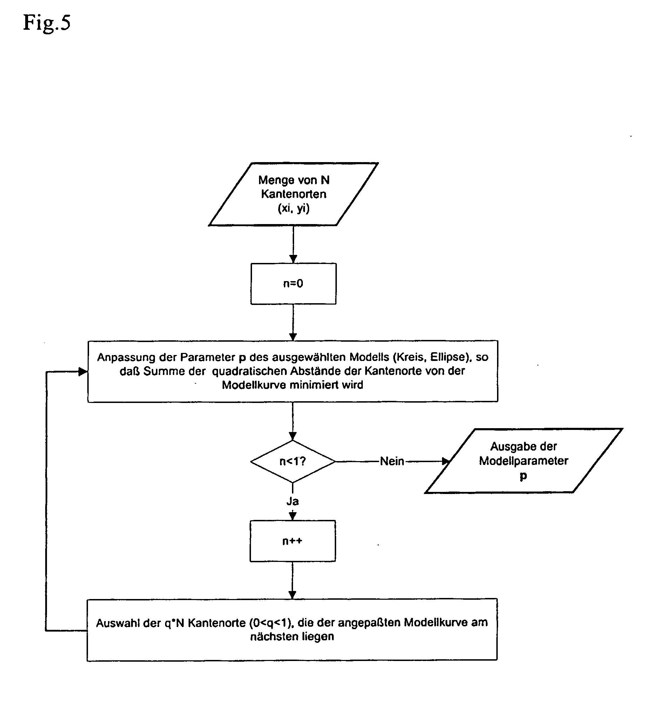

**Anpassung des Pupillen- / Irismodells** (**Fig. 5**)

Aus der Menge der im vorhergehenden Schritt bestimmten Kantenorte ($x_i$,yi) können die Parameter des anzupassenden Pupillen- und Irismodells (also entweder Kreis oder Ellipse) durch Regression bestimmt werden. Dies geschieht durch die Minimierung der Summe quadratischer Fehler

$$\Sigma_i \ (x_i - x(x_i, y_i, \mathbf{p}))^2 + (y_i - y(x_i, y_i, \mathbf{p}))^2 \rightarrow min \qquad\qquad (1)$$

über die Menge möglicher Parametervektoren **p** (Kreis: Mittelpunktskoordinaten und Radius, Ellipse: Mittelpunktskoordinaten, Längen der Hauptachsen, Winkel der großen Hauptachse zur x-Achse). Für die Kreisanpassung ist eine Lösung von (1) unmittelbar und numerisch effizient mit dem Verfahren der Singulärwertzerlegung möglich. Für die Lösung des restringierten Quadratmittelproblems zur Ellipsenanpassung existieren ebenfalls eine Reihe von Standardansätzen (z.B. in Bookstein F.L., "Fitting conic sections to scattered data", Computer Graphics and image Processing, Vol. 9, S. 56-71, 1979, und Fitzgibbon A.W. and Fisher R.B., "A buyer's guide to conic fitting", Proceedings of British Machine Vision Conference, Birmingham, 1995).

Zur Reduktion des Einflusses von Ausreißern (d.h., falsch bestimmten Kantenorten) wird ein zweistufiges Regressionsverfahren nach Fig. 5 verwendet.

Außerdem können alternative Verfahren zur Selektion der Kantenorte, die zur Parameteranpassung verwendet werden sollen, genutzt werden, wie beispielsweise die Hough-Transformation für Kreismodelle.

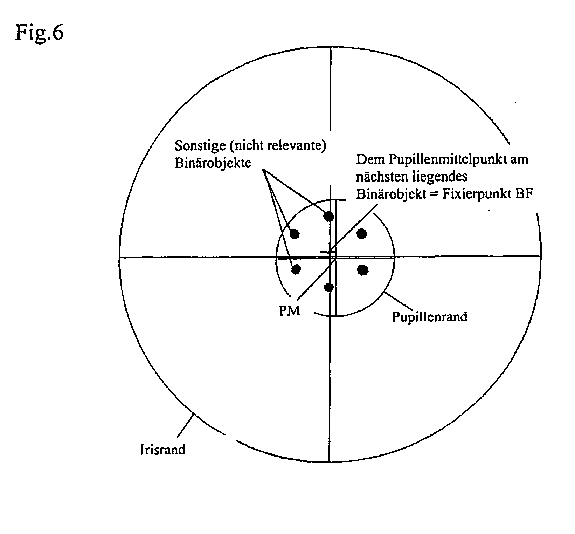

### *Detektion des Fixierpunktes* (*Fig. 6*)

Zur Detektion des Fixierpunktes erfolgt eine Binarisierung (siehe oben) des ungefilterten Bildes der CCD Kamera mit einem von thr* (Schwellwert, der zur Grobdetektion der Pupille benutzt wurde) abhängigen Schwellwert $\Theta_{FP}$ = s*thr* (s>1.0). Als Fixierpunkt wird der Mittelpunkt des dem bestimmten Pupillenmittelpunkt PM am nächsten liegenden zu-sammenhängenden Binärobjektes BF (Grauwerte > $\Theta_{FP}$) bestimmt, das eine bestimmte Mindestfläche aufweist.

Sonstige nicht relevante Binärobjekte (z.B. Reflexbilder der LED Beleuchtung) werden anhand ihrer größeren Entfernung vom Pupillenmittelpunkt identifiziert und bleiben unberücksichtigt.

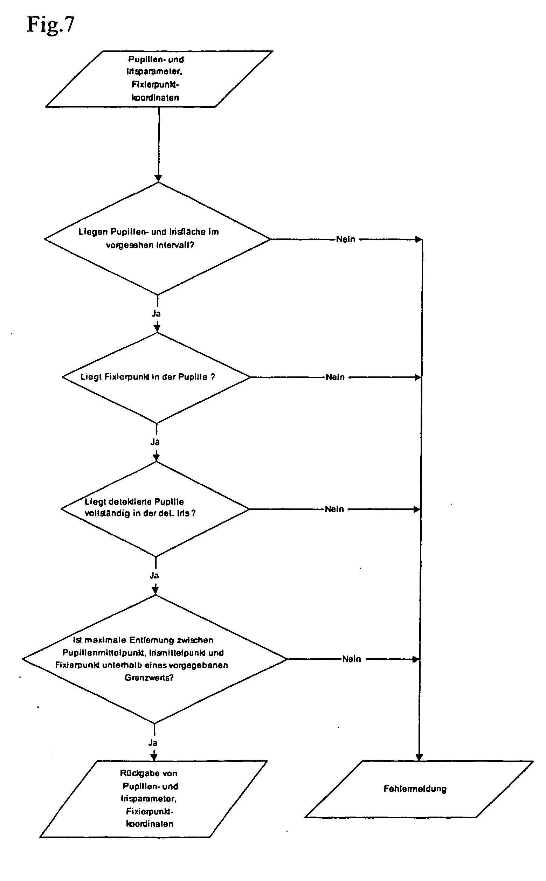

### *Plausibilitätskontrolle* (*Fig.7*)

Vor der Rückgabe der ermittelten Koordinaten an das aufrufende Programm wird eine Plausibilitätskontrolle nach Fig. 7 vorgenommen, um zu verhindern, daß eventuell unrichtig detektierte Elemente gefunden wurden. Die Abfragen beinhalten vorher bekannte Eigenschaften des untersuchten Objektes, mit welchen die ermittelten Ergebnisse in Koinzidenz stehen müssen.

### Patentansprüche

1. Verfahren zur Ermittlung von Abständen am vorderen Augenabschnitt, vorzugsweise des Irisdurchmessers, wobei mittels einer Bildaufnahmeeinheit und einer Anordnung zur Beleuchtung des Auges das Bild mindestens eines Teiles des Auges erfaßt und digitalisiert wird, aus dem digitalisierten Bild mittels einer Intensitäts-Schwellenanalyse und einer Schwerpunktanalyse eine Grobbestimmung des Pupillenmittelpunktes erfolgt und anhand der Grobbestimmung eine Feindetektion der Orte der Iriskanten durchgeführt wird, wobei die Feindetektion umfaßt:

   - Abtasten des digitalisierten Bildes entlang mehrerer Geraden, die jeweils durch den bei der Grobbestimmung ermittelten Pupillenmittelpunkt laufen, wobei die Orte der Iriskante entlang jeder Gerade detektiert werden, wobei jede Gerade für die Detektion der Iriskante mit einer horizontalen Achse durch die Pupille jeweils einen Winkel $\alpha$ einschließt, und beim Abtasten die Richtung der Geraden und damit der Winkel $\alpha$ für die Geraden sukzessive geändert wird, und
   - Modellieren der Iris mittels eines kreisförmigen oder elliptischen Kantenmodells der Iris auf Basis der zuvor detektierten Orte der Kante,
   - wobei bei allen Geraden der Winkel $\alpha$ für die Detektion der Iriskante gemäß $\alpha_{min, Iris} < \alpha < \alpha_{max, Iris}$ eingeschränkt ist und damit die Kantenbestimmung nur in zwei Kreissektoren um den Pupillenmittelpunkt erfolgt.

2. Verfahren nach Anspruch 1, wobei zusätzlich aus der Lage eines Fixierreflexes der Durchstoßpunkt der Sehachse durch die Augenhornhaut in Bezug auf die Pupille und/oder Iris ermittelt wird.

3. Verfahren nach einem der Ansprüche 1 oder 2, wobei aus der Lage eines Fixierreflexes zum Pupillenmittelpunkt und/oder Irismittelpunkt der Winkel zwischen Sehachse und optischer Achse des Auges bestimmt wird.

4. Verfahren nach Anspruch 3, wobei mittels des Winkels ein Gerät zur interferometrischen Vermessung von Teilabschnitten des menschlichen Auges entlang der optischen Achse dieses Auges voreingestellt wird.

5. Verfahren nach einem der obigen Ansprüche, wobei auch der Pupillendurchmesser ermittelt wird, indem die Feindetektion weiter umfaßt

   - beim Abtasten des digitalisierten Bildes entlang der mehreren Geraden, die jeweils durch den bei der Grobbestimmung ermittelten Pupillenmittelpunkt laufen, werden die Orte der Pupillenkante entlang jeder der Geraden detektiert, die mit der horizontalen Achse durch die Pupille jeweils einen Winkel $\alpha$ einschließen und deren Winkel $\alpha$ beim Abtasten sukzessive geändert wird, und
   - Modellieren der Pupille mittels eines kreisförmigen oder elliptischen Kantenmodells der Pupille auf Basis der zuvor detektierten Orte der Kante,
   - wobei bei allen Geraden der Winkel $\alpha$ für die Detektion der Pupillenkante gemäß $\alpha_{min, Pupille} < \alpha < \alpha_{max, Pupille}$ eingeschränkt ist und damit die Kantenbestimmung für die Pupille ebenfalls nur in zwei Kreissektoren um den Pupillenmittelpunkt erfolgt.

**Claims**

1. A method for determining distances in the anterior ocular segment, preferably the iris diameter, wherein the image of at least part of the eye is captured and digitized using an image-capturing unit and an arrangement for illumination of the eye, the pupil center is roughly detected by means of a centroid analysis and an intensity threshold analysis from the digitized image and a fine detection of the positions of the iris edges is effected on the basis of said rough detection, said fine detection comprising:

   - scanning the digitized image along several straight lines, each of said straight lines extending through the pupil center determined by the rough detection, wherein the positions of the iris edge are detected along each straight line, wherein each straight line used for detection of the iris edge encloses an angle $\alpha$ with a horizontal axis through the pupil, and the direction of the straight lines and, thus, the angle $\alpha$ for the straight lines is successively changed during scanning, and
   - modeling the iris by means of a circular or elliptic edge model of the iris on the basis of the previously detected positions of the edge,
   - wherein the angle $\alpha$ is limited for detection of the iris edge for all straight lines in accordance with $\alpha_{min, Iris} < \alpha < \alpha_{max, Iris}$ and, thus, the edge determination is effected in only two circular sectors around the pupil center.

2. Method according to claim 1, wherein, in addition, the intersection point of the visual axis through the cornea of the eye relative to the pupil and/or the iris is determined on the basis of the position of a fixation reflex.

3. Method according to any one of claims 1 or 2, wherein the angle between the visual axis and the optical axis of the eye is determined on the basis of the position of a fixation reflex relative to the pupil center and/or iris center.

4. Method according to claim 3, wherein an instrument for interferometric measurement of segments of the human eye is preadjusted along the optical axis of said eye using said angle.

5. Method according to any one of the above claims, wherein further the pupil diameter is determined by the fine detection further comprising:

   - during scanning of the digitized image along several straight lines, each of said straight lines extending through the roughly detected pupil center, the positions of the pupil edge are detected along each straight line, wherein each straight line encloses an angle $\alpha$ with the horizontal axis through the pupil and wherein this angle $\alpha$ is successively changed during scanning, and
   - modeling the pupil by means of a circular or elliptic edge model of the pupil on the basis of the previously detected positions of the edge,

- wherein the angle $\alpha$ is limited for detection of the pupil edge for all straight lines in accordance with $\alpha_{min, Pupille} < \alpha < \alpha_{max, Pupille}$ and, thus, the edge determination for the pupil is also effected in only two circular sectors around the pupil center.

**Revendications**

1. Procédé pour la détermination d'intervalles dans la partie avant de l'oeil, de préférence du diamètre de l'iris, dans lequel l'image d'au moins une partie de l'oeil est saisie et numérisée au moyen d'une unité de prise de vue et d'un dispositif d'éclairage de l'oeil, une localisation grossière du centre de la pupille est effectuée à partir de l'image numérisée par une analyse de seuil d'intensité et une analyse de centre de gravité et une détection fine des positions du bord de l'iris est effectuée à partir de la localisation grossière, la détection fine comprenant :

   - l'exploration de l'image numérisée le long de plusieurs droites passant chacune par le centre de la pupille déterminé par la localisation grossière, les positions du bord de l'iris étant détectées le long d'une droite, chaque droite faisant chaque fois, pour la détection du bord de l'iris, un angle $\alpha$ avec un axe horizontal traversant la pupille et, lors de l'exploration, la direction de la droite, et de ce fait l'angle $\alpha$ avec les droites étant successivement modifié et
   - la modélisation de l'iris au moyen d'un modèle de bord circulaire ou elliptique de l'iris sur la base des positions du bord précédemment détectées,
   - l'angle $\alpha$ pour la détection du bord de l'iris étant limité pour toutes les droites par la fonction $\alpha_{min, iris} < \alpha < \alpha_{max, iris}$ et la détermination du bord n'étant ainsi effectuée que dans deux secteurs de cercle autour du centre de la pupille.

2. Procédé selon la revendication 1, dans lequel on détecte en outre à partir de la position d'un réflexe de fixation, le point de passage de l'axe de vision à travers la cornée par rapport à la pupille et/ou l'iris.

3. Procédé selon la revendication 1 ou 2, dans lequel on détermine à partir de la position d'un réflexe de fixation par rapport au centre de la pupille et/ou au centre de l'iris, l'angle entre l'axe de vision et l'axe optique de l'oeil

4. Procédé selon la revendication 3, dans lequel un appareil pour faire des mesures interférométriques de sections partielles de l'oeil humain le long de l'axe optique de cet oeil est préréglé au moyen de l'angle.

5. Procédé selon l'une des revendications précédentes, dans lequel on détermine aussi le diamètre de la pupille par le fait que la détection fine comprend en outre :

   - la détection, lors de l'exploration de l'image numérisée le long des nombreuses droites qui passent chacune par le centre de la pupille grossièrement localisée, des positions du bord de la pupille le long de chaque droite qui forment chaque fois un angle $\alpha$ avec l'axe horizontal passant par la pupille et dont l'angle $\alpha$ est modifié successivement lors de l'exploration et
   - la modélisation de la pupille au moyen d'un modèle de bord circulaire ou elliptique de la pupille sur la base des positions du bord précédemment détectées,
   - l'angle $\alpha$ pour la détection du bord de la pupille étant limité pour toutes les droites selon la fonction $\alpha_{min, pupille} < \alpha < \alpha_{max, pupille}$ et la détermination du bord pour la pupille n'étant ainsi effectuée que dans deux secteurs de cercle autour du centre de la pupille.

Fig.1

```
┌─────────────────────────────┐
│      digitalisierte         │
│    Aufnahme mit             │
│   Umfeldbeleuchtung         │
└─────────────────────────────┘
              │
              ▼
┌─────────────────────────────┐
│     Rauschunterdrückung     │
└─────────────────────────────┘
              │
              ▼
┌─────────────────────────────┐
│    Grobdetektion der Pupille│
└─────────────────────────────┘
              │
              ▼
┌─────────────────────────────┐
│     Feindetektion der       │
│       Pupillenkante         │
└─────────────────────────────┘
              │
              ▼
┌─────────────────────────────┐
│  Feindetektion der Iriskante│
└─────────────────────────────┘
              │
              ▼
┌─────────────────────────────┐
│    Dektion der Sehachse     │
│    (Fixierpunktreflex)      │
└─────────────────────────────┘
              │
              ▼
┌─────────────────────────────┐
│    Plausibilitätskontrolle  │
└─────────────────────────────┘
              │
              ▼
┌─────────────────────────────┐
│    Pupillen-, Iris- und     │
│        Sehachsen-           │
│        parameter            │
└─────────────────────────────┘
```

# Fig.2

```
┌─────────────────┐
│   thr = thr_0   │
└────────┬────────┘
         │
         ▼
┌─────────────────┐
│  ˙ Binärbild =  │◄──────────────┐
│   (Bild < thr)  │               │
└────────┬────────┘               │
         │                        │
         ▼                        │
┌─────────────────────┐    ┌──────────┐
│ Suche dem           │    │  thr ++  │
│ Bildmittelpunkt am  │    └────┬─────┘
│ nächsten liegendes  │         │
│ Binärobjekt mit     │         │
│ A > A_min :         │         │
│ BObj(thr)           │         │
└────────┬────────────┘         │
         │                      │
         ▼                      │
      ◇──────────◇              │
     ╱ thr =      ╲──NEIN───────┘
     ╲ thr_max?  ╱
      ◇──────────◇
         │
         J
         A
         ▼
┌──────────────────────────────────────────┐
│          optimale Schwelle                │
│ thr* := |Bobj(thr*)-BObj(thr*+1)| -> min  │
└────────────────────┬─────────────────────┘
                     │
                     ▼
┌──────────────────────────────────────────┐
│ (x0,y0) := Mittelpunkt(BObj(thr*))        │
│ r0 := Fläche(BObj(thr*))/PI               │
└──────────────────────────────────────────┘
```

Fig.3

$g(x,y) = 1$

$(x0, y0)$

R

y

x

Fig.4

Strecke, entlang welcher Grauwerte im
gefilterten Bild abgetastet werden

GP

S

SB

für Kantenort

Suchbereich

$(x_0, y_0)$

α

K
Pupille bzw. Iris

Fig.5

Menge von N
Kantenorten
(xi, yi)

n=0

Anpassung der Parameter p des ausgewählten Modells (Kreis, Ellipse), so
daß Summe der quadratischen Abstände der Kantenorte von der
Modellkurve minimiert wird

n<1? —Nein→ Ausgabe der
Modellparameter
p

Ja

n++

Auswahl der q*N Kantenorte (0<q<1), die der angepaßten Modellkurve am
nächsten liegen

Fig.6

Sonstige (nicht relevante)
Binärobjekte

Dem Pupillenmittelpunkt am
nächsten liegendes
Binärobjekt = Fixierpunkt BF

PM

Pupillenrand

Irisrand

## Fig.7

Pupillen- und Irisparameter, Fixierpunkt-koordinaten

Liegen Pupillen- und Irisfläche im vorgesehen Intervall? — Nein

Ja

Liegt Fixierpunkt in der Pupille ? — Nein

Ja

Liegt detektierte Pupille vollständig in der det. Iris ? — Nein

Ja

Ist maximale Entfernung zwischen Pupillenmittelpunkt, Irismittelpunkt und Fixierpunkt unterhalb eines vorgegebenen Grenzwerts? — Nein

Ja

Rückgabe von Pupillen- und Irisparameter, Fixierpunkt-koordinaten

Fehlermeldung

Fig. 8 schematische Darstellung der erfindungsgemäßen Anordnung

Fig. 9 gegrabbtes Bild eines zu vermessenden Auges

Fig. 10 Vergrößerung des zentralen Teiles von Fig. 9

Fig.11 Weiß-zu-Weiß-Schablone nach Holladay-Godwin:

AE-1576 Holladay-Godwin
Cornea Guage

**IN DER BESCHREIBUNG AUFGEFÜHRTE DOKUMENTE**

**In der Beschreibung aufgeführte Patentdokumente**

- US 4319564 A
- US 4398812 A
- EP 0550673 A

- US 5291234 A
- US 5469234 A
- WO 0033729 A

**In der Beschreibung aufgeführte Nicht-Patentliteratur**

- **JACK T. HOLLADAY.** Intraocular Lens Power Calculations for the Refractive Surgeon. *Operative Techniques in Cataract and Refractive Surgery,* September 1998, vol. 1 (3), 105-117
- **SAVITZKY A. ; GOLAY, M.J.E.** *Analytical Chemistry,* 1964, vol. 36, 1627-39

- **BOOKSTEIN F.L.** Fitting conic sections to scattered data. *Computer Graphics and image Processing,* 1979, vol. 9, 56-71
- **FITZGIBBON A.W. ; FISHER R.B.** A buyer's guide to conic fitting. *Proceedings of British Machine Vision Conference,* 1995